# FASCICULE DE BREVET EUROPEEN

(11) **EP 0 533 562 B1**
(45) Date de publication et mention de la délivrance du brevet: **22.01.1997**
(21) Numéro de dépôt: 92402535.6
(22) Date de dépôt: 16.09.1992
(51) Int. Cl.: G01N 33/28, G01N 25/02

(54) **Dispositif pour la détection de l'apparition ou de la disparition de deux phases dans un produit hydrocarboné liquide**
Vorrichtung zum Nachweis des Auftretens oder Verschwindens zweier Phasen in einer flüssigen Kohlenwasserstoff-Substanz
Device for ascertaining the appearance or disappearance of two phases in a fluid hydrocarbon substance

(30) Priorité: 17.09.1991 FR 9111442
(43) Date de publication de la demande: 24.03.1993
(73) Titulaire: TOTAL RAFFINAGE DISTRIBUTION S.A., 92800 Puteaux (FR)
(72) Inventeur: Dutot, Philippe, F-76280 Fongueuesmare (FR)
(74) Mandataire: Jolly, Jean-Pierre

(56) Documents cités:
- FR-A- 1 417 250
- GB-A- 966 828
- US-A- 3 646 313
- US-A- 4 066 365
- US-A- 4 804 274
- PATENT ABSTRACTS OF JAPAN, vol. 14, no. 349 (P-1084)(4292), 27 juillet 1990
- PATENT ABSTRACTS OF JAPAN, vol. 10, no. 382 (P-529)(2439), 20 décembre 1986

## Description

La présente invention concerne un dispositif pour la détection de l'apparition ou de la disparition de deux phases dans un produit hydrocarboné liquide, notamment une coupe prétrolière.

De nombreux mélanges se présentent sous le forme d'une unique phase liquide, dans certaines conditions de température et de pression, et de deux phases distinctes dans d'autres conditions. Ces deux phases peuvent être des liquides non miscibles, ou un liquide et au moins un solide, résultant de la précipitation ou de la cristallisation d'au moins une espèce chimique dans le liquide, et elles se fondent en une unique phase liquide lorsque les conditions appropriées de température et de pression sont réunies.

La connaissance de la température de transition correspondant au début de la miscibilité ou de l'immiscibilité des deux phases présente un grand intérêt, notamment pour les mélanges hydrocarbonés d'origine pétrolière, et on la détermine en observant l'apparition ou la disparition d'un léger trouble dans le mélange, caractéristique de la présence de certains composés.

Ces températures critiques seront appelées "point de trouble" dans la suite de la présente description, où l'on se référera plus spécifiquement à des points de trouble particuliers, mesurés notamment sur des mélanges hydrocarbonnés d'origine pétrolière, à savoir le point d'aniline (voir normes ISO 2977, ASTM D611 et AFNOR NFM 07-021), ou encore le point de trouble des produits pétroliers (déterminé par la norme AFNOR NF T 60-105), ou encore le point de décongélation. Ces divers points de trouble permettent de caractériser les hydrocarbures purs et d'analyser les mélanges d'hydrocarbures, en fonction de la température, entre - 60 et + 100°C.

Parmi ces points de trouble, le point d'aniline est défini comme étant la température minimum, en degrés C, d'une solution à l'équilibre, pour des volumes égaux d'aniline et du produit testé. Les hydrocarbures aromatiques présentent les points d'aniline les plus faibles, tandis que les hydrocarbures paraffiniques présentent les points d'aniline les plus élevés, les cycloparaffines et les oléfines présentant des points d'aniline intermédiaires. La mesure du point d'aniline d'un produit pétrolier est donc un moyen fréquemment utilisé pour estimer qualitativement la teneur de ce produit en hydrocarbures aromatiques.

La mesure des points d'aniline est une opération relativement complexe et c'est ainsi que la norme ISO 2977 décrit cinq méthodes différentes, suivant la coloration des échantillons traités et suivant la nature de la mesure, manuelle ou automatique. Des précautions sérieuses doivent, en effet, être prises pour effectuer ces mesures, du fait de l'extrême toxicité de l'aniline.

C'est ainsi, par exemple, que l'on a proposé d'utiliser des tubes scellés pour des produits transparents et volatils, des tubes ouverts pour des produits clairs et peu volatils et une technique en couche mince pour des produits colorés non volatils.

Le point de trouble des produits pétroliers, notamment de carburants ou de combustibles liquides, est défini, lorsque l'on refroidit ces produits, comme étant la température à laquelle apparaissent au sein du liquide les premiers cristaux des composés ayant le point de congélation le plus élevé.

Le troisième point de trouble, au sens de l'invention, est le point de décongélation des carburateurs. Ce point de décongélation est déterminé, selon la norme NFM 0 7048, en soumettant un échantillon de carburant à un cycle alterné de refroidissement, entraînant l'apparition de cristaux, et de réchauffement, entraînant la disparition de ces cristaux. La température à laquelle disparaissent les derniers cristaux est appelée température de décongélation.

US-A-4 804 274 décrit une méthode et un appareil pour la détermination du point de trouble, du point d'écoulement et/ou du point de congélation de carburants hydrocarbonés, ou de carburants hydrocarbonés et oxygénés.

L'appareil décrit dans ce brevet antérieur comprend un récipient tubulaire, un moyen de mesure de la température du mélange à étudier, un moyen d'obturation, un bloc apte à contenir le récipient tubulaire, un agitateur magnétique, un socle équipé de moyens permettant d'imprimer un mouvement rotatoire au mélange par l'intermédiaire de l'agitateur, et des moyens d'émission et de réception d'un faisceau lumineux.

Cet appareil ne possède cependant pas de moyens intrinsèques de chauffage ou de refroidissement de l'échantillon traité. En outre et surtout, le récipient tubulaire de cet appareil est vertical, ce qui nuit à la migration de la phase gazeuse.

La présente invention vise à proposer un appareil pour la détection de l'apparition ou de la disparition de deux phases dans un produit hydrocarboné liquide, notamment du point d'aniline, du point de trouble et du point de décongélation d'une charge hydrocarbonée, qui puisse être utilisé plus facilement que les dispositifs de la technique antérieure et qui ait un caractère universel, c'est-à-dire qui puisse être appliqué quelles que soient la coloration du produit testé et la température correspondant au point de trouble.

Un premier but de l'invention est donc de proposer un appareil pour la détection automatique de l'apparition ou de la disparition de deux phases dans un produit hydrocarboné liquide, qui puisse être utilisé quelle que soit la coloration du produit traité.

Un autre but de l'invention est de proposer un dispositif de ce type, qui soit applicable à la mesure de points de trouble compris entre - 60 et + 100°C, et même au-delà.

L'invention a aussi pour but de proposer un appareil de ce type, dans lequel les risques de mise en contact de l'opérateur avec les produits présents et notamment avec l'aniline sont extrêmement réduits.

L'invention a enfin pour but de proposer un dispositif de ce type, dans lequel l'apparition ou la disparition de deux phases ne fait pas appel à la vision de l'opérateur, mais est détectée par des moyens de mesure beaucoup plus sensibles que l'oeil humain.

A cet effet, l'invention a pour objet un dispositif pour la détection de l'apparition et de la disparition de deux phases dans un produit liquide hydrocarboné, caractérisé en ce qu'il comprend :
- un récipient tubulaire apte à contenir le mélange des deux phases liquides, ce récipient étant équipé d'un moyen de mesure de la température du mélange et d'un moyen d'obturation hermétique :
- une ossature rigide comprenant un logement apte à recevoir ledit récipient ;
- un moyen de réglage de la température dudit logement ;
- des moyens d'émission et de réception d'un faisceau optique , disposés transversalement, de part et d'autre dudit logement ; et
- un socle destiné à supporter l'ossature, ce socle étant équipé de moyens aptes à lui imprimer des mouvements vibratoires ;

ce dispositif étant caractérisé en ce que le logement destiné à recevoir le récipient est incliné d'un angle compris entre 10 et 45° et, de préférence, entre 20 et 25° par rapport à l'horizontale.

Le récipient contenant le mélange d'hydrocarbures sera, par exemple, un flacon tubulaire, fermé par une capsule métallique munie d'une pastille d'obturation en élastomère, à travers laquelle passe une sonde de mesure de température, comprenant par exemple un thermocouple. Ce flacon, rempli du mélange à tester, ne sera pas ouvert en cours de mesure et l'opérateur sera ainsi parfaitement isolé de son contenu. L'échantillon à tester sera d'un très faible volume, de l'ordre, par exemple, de 1,5 ml.

L'homme du métier pourra, par exemple, utiliser ce flacon, contenant de l'aniline préinjectée, pour mesurer le point d'aniline d'un produit, sans le moindre risque de contact avec l'aniline.

L'ossature comportant un logement pour le récipient sera constituée, par exemple, d'un bloc métallique, percé d'un évidement tubulaire de forme complémentaire de celle du flacon contenant l'échantillon à tester. Ce bloc métallique comprendra par exemple une résistance chauffante et un circuit de réfrigération à circulation de fluide caloporteur. Le bloc métallique pourra aussi être équipé d'un système de chauffage et de refroidissement à effet PELTIER.

Ainsi qu'il a été indiqué, le logement du récipient conforme à l'invention est incliné d'un angle compris entre 10 et 45° et, de préférence, de 20 à 25°, par rapport à l'horizontale. Ainsi, la phase gazeuse nécessaire à une agitation efficace du produit à tester migrera avantageusement dans la partie haute du récipient. Ce logement pourra être disposé horizontalement dans l'ossature et celle-ci reposera sur une surface inclinée du support apte à comprimer des vibrations à cette ossature. L'ossature pourra aussi reposer sur une surface horizontale de ce support, le logement étant alors incliné par rapport à l'horizontale dans cette ossature.

Le dispositif conforme à l'invention est donc d'une grande simplicité et se prête à des mesures faciles et en serie du point de trouble de nombreux échantillons, en particulier du point d'aniline d'une charge hydrocarbonée, telle qu'un produit pétrolier ou un solvant hydrocarboné.

Une forme de réalisation de l':invention va être décrite ci-après, à titre d'exemple non limitatif, en référence aux dessins schématiques annexés. Sur ces dessins :

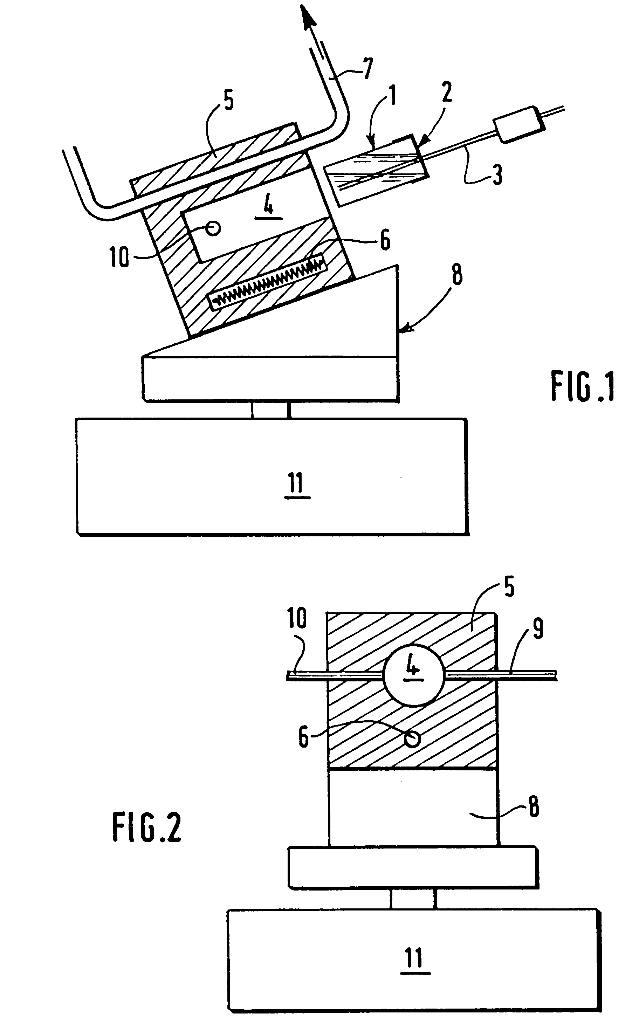

- la figure 1 est une vue en élévation latérale, partiellement en coupe, du dispositif;
- la figure 2 est une vue de bout, partiellement en coupe, du dispositif de la figure 1 ;

Sur ces dessins, le récipient est désigné par la référence 1. Il a une forme tubulaire et il est obturé à son extrémité libre par une capsule en aluminium 2, munie d'une pastille d'obturation en élastomère, à travers laquelle est engagée une sonde 3, par exemple à thermocouple NiCr/NiAl, destinée à mesurer la température du liquide présent dans le récipient 1.

Aux fins de la mesure, ce récipient est logé dans un évidement 4 , de forme complémentaire, d'un bloc métallique 5, comportant une résistance chauffante 6 et un circuit 7 de réfrigération, à circulation d'un fluide caloporteur. Tout autre mode de chauffage et de réfrigération, par effet PELTIER, par exemple, pourrait être utilisé.

Comme représenté, l'évidement 4 du bloc métallique 5 peut avoir un axe normalement disposé à l'horizontale et le bloc 5 repose alors en cours de mesure sur la face supérieure inclinée d'un support 8. Cette face, inclinée par exemple d'un angle de 20° par rapport à l'axe du récipient 1 et du logement 4, permet à la phase gazeuse présente dans le récipient 1 de se rassembler à la partie supérieure de celui-ci et de ne pas interférer avec les mesures effectuées. La surface supérieure du support 8 pourrait également être horizontale, avec l'évidement 8 incliné dans ce cas dans le bloc 5 par rapport à l'horizontale.

Les mesures sont conduites par exemple à l'aide d'un faisceau lumineux acheminé par une fibre optique 9, connectée à un dispositif émetteur du faisceau et débouchant latéralement dans le logement 4, en regard de l'extrémité d'une fibre optique 10, connectée à un système de mesure de l'intensité lumineuse. Les deux fibres 9 et 10 déboucheront dans le logement 4 au voisinage de son extrémité fermée et seront par conséquent éloignées de la phase gazeuse rassemblée à la partie supérieure de l'autre extrémité du récipient 1. Il sera possible, ainsi, d'effectuer des mesures précises de l'intensité lumineuse reçue par la fibre 10 à travers le liquide, en fonction des variations de température provoquées par la résistance chauffante 6 et par le circuit de réfrigération 7, variations que mesurera la sonde de température 3.

Comme faisceau lumineux, on utilisera avantageusement un rayonnement infrarouge, par exemple de longueur d'onde égale à 880 nm. Le support 8 est solidaire d'un dispositif vibrant 11, apte à imprimer au bloc métallique 5 des vibrations suffisantes pour homogénéiser le mélange des liquides contenus dans le récipient 1.

On notera que le remplissage du récipient 1 peut s'effectuer à l'aide d'une seringue à travers le joint élastomère de la capsule métallique 2, en dehors du local de mesure, où les échantillons à tester pourront ensuite être transférés et manipulés sans aucun risque par l'opérateur.

La fiabilité de cet appareillage a été confirmée en éffectuant des essais comparatifs de mesure du point d'aniline sur un grand nombre de produits, avec le dispositif qui vient d'être décrit, d'une part, et selon les méthodes manuelles décrites dans la norme NF M 07 021, d'autre part. Ces essais ont porté sur des mélanges de volumes égaux d'aniline et des produits testés, mélanges réalisés avec une précision relative inférieure à 1 % à partir des masses volumiques des constituants du mélange.

Les résultats des mesures effectuées apparaissent dans le Tableau ci-après. Ces résultats confirment la fiabilité du dispositif conforme à l'invention.

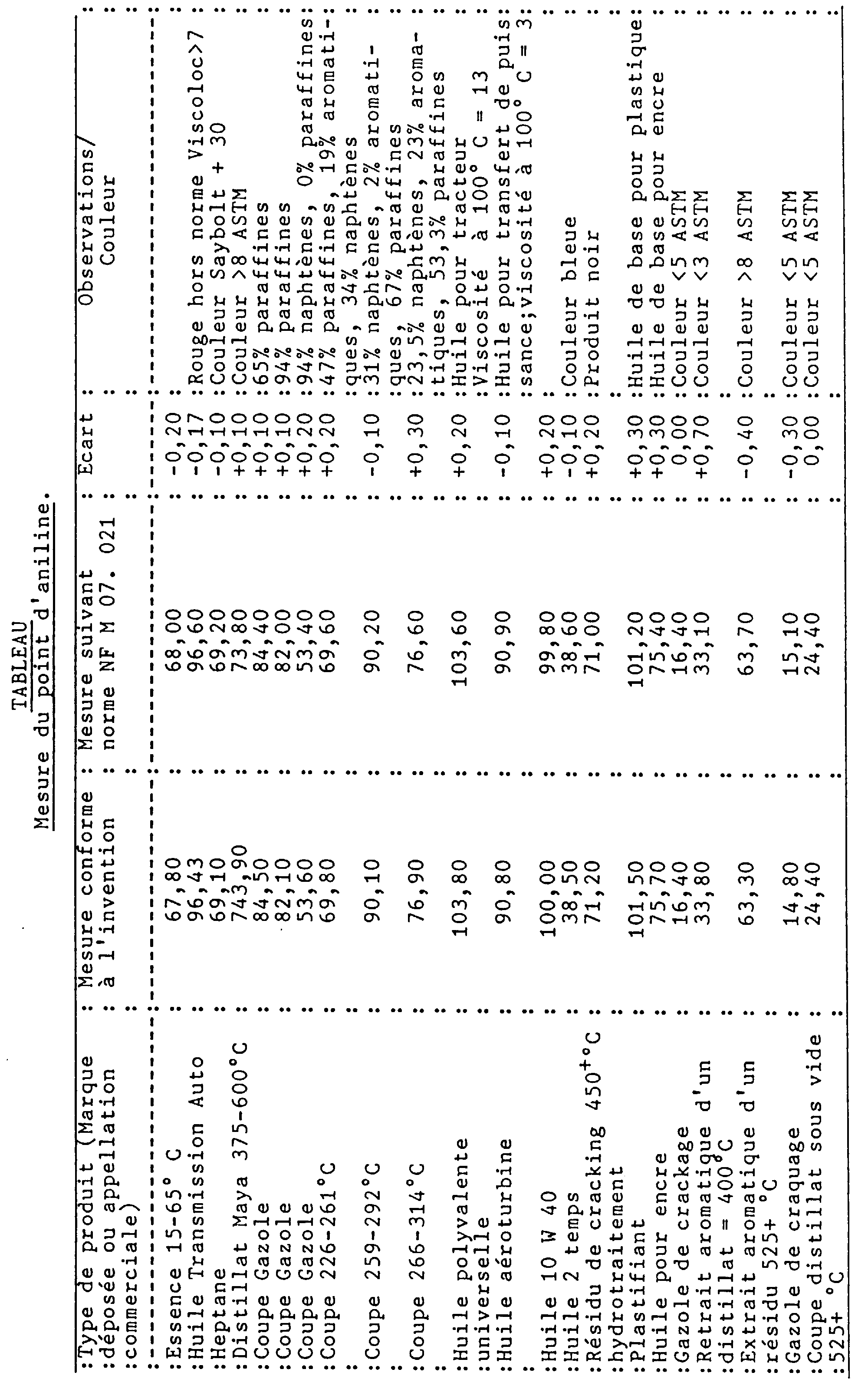

L'écart entre les résultats de la méthode usuelle normalisée et ceux de la méthode selon l'invention est toujours inférieur à la reproductibilité reconnue dans la technique normalisée.

On notera qu'une fois les échantillons préparés, leur manipulation et les mesures effectuées par le dispositif conforme à l'invention peuvent être menées de manière entièrement automatique, sans intervention de l'opérateur.

## Revendications

1. Dispositif de détection de l'apparition ou de la disparition de deux phases dans un produit hydrocarboné liquide, ce dispositif comprenant :
- un récipient tubulaire (1) apte à contenir le mélange des deux phases, ce récipient étant équipé d'un moyen (3) de mesure de la température du mélange et d'un moyen (2) d'obturation hermétique :
- une ossature rigide (5), comprenant un logement (4) apte à recevoir le récipient (1) ;
- un moyen de réglage (5,6) de la température du logement (4) ;
- des moyens d'émission (9) et de réception (10) d'un faisceau lumineux, disposés transversalement de part et d'autre dudit logement ; et
- un socle (8) destiné à supporter l'ossature (5), ce socle étant équipé de moyens (11) aptes à lui imprimer des mouvements vibratoires ;
ce dispositif étant caractérisé en ce que le logement (4) destiné à recevoir le récipient (1) est incliné d'un angle compris entre 10 et 45° et, de préférence, entre 20 et 25° par rapport à l'horizontale.

2. Dispositif selon la revendication 1, caractérisé en ce que le récipient tubulaire (1) est fermé par une capsule métallique (2) munie d'une pastille d'obturation en élastomère, à travers laquelle passe une sonde (3) de mesure de température.

3. Dispositif selon l'une des revendications 1 et 2, caractérisé en ce que l'ossature (5) comprend un bloc métallique percé d'un évidement tubulaire (4) de forme complémentaire de celle du récipient (1).

4. Dispositif selon la revendication 3, caractérisé en ce que le bloc métallique (5) comprend une résistance chauffante (6) et un circuit de réfrigération (7) à circulation d'un fluide caloporteur.

5. Dispositif selon la revendication 3, caractérisé en ce que le bloc métallique (5) comprend un système de chauffage et de réfrigération à effet PELTIER.

6. Dispositif selon l'une des revendications 1 à 5, caractérisé en ce que ledit faisceau optique est un faisceau de rayonnement infrarouge.

## Patentansprüche

1. Vorrichtung zur Feststellung des Auftretens oder des Verschwindens zweier Phasen in einem flüssigen Kohlenwasserstoffprodukt, welche
einen rohrförmigen Behälter (1) zur Aufnahme des Gemisches der beiden Phasen, der mit einem Mittel (3) zur Messung der Temperatur des Gemisches und mit einem Mittel (2) zum hermetischen Verschließen versehen ist,
ein starres Gerüst (5) mit einem Raum (4) zur Aufnahme des Behälters (1),
ein Mittel (6, 7) zur Regulierung der Temperatur des Raumes (4),
Mittel (9, 10) zum Ausstrahlen und zum Empfangen eines Lichtstrahls, welche quer beiderseits des Raumes (4) angeordnet sind, und
einen Sockel (8) zur Abstützung des Gerüstes (5), der mit Mitteln (11) versehen ist, um ihn in Schwingungen zu versetzen,
aufweist, dadurch **gekennzeichnet,** daß der Raum (4) zur Aufnahme des Behälters (1) um einen Winkel zwischen 10° und 45°, vorzugsweise zwischen 20° und 25°, gegenüber der Horizontalen geneigt ist.

2. Vorrichtung nach Anspruch 1, dadurch **gekennzeichnet,** daß der rohrförmige Behälter (1) durch eine mit einer Verschlußscheibe aus einem Elastomeren versehene Metallkapsel (2) verschlossen ist, durch welche eine Temperaturmeßsonde (3) hindurchtritt.

3. Vorrichtung nach Anspruch 1 oder 2, dadurch **gekennzeichnet,** daß das Gerüst (5) aus einem Metallblock besteht, welcher mit einer rohrförmigen Ausnehmung (4) versehen ist, deren Gestalt komplementär zu derjenigen des Behälters (1) ist.

4. Vorrichtung nach Anspruch 3, dadurch **gekennzeichnet,** daß der Metallblock (5) einen Heizwiderstand (6) und einen Kühlkreis (7) aufweist, in welchem ein wärmeabführendes Strömungsmittel strömt.

5. Vorrichtung nach Anspruch 3, dadurch **gekennzeichnet,** daß der Metallblock (5) ein auf dem Peltier-Effekt basierendes Heiz- und Kühlsystem aufweist.

6. Vorrichtung nach einem der Ansprüche 1 bis 5, dadurch **gekennzeichnet,** daß es sich beim optischen Strahl um einen Strahl infraroter Strahlung handelt.

## Claims

1. A device for detecting the appearance or disappearance of two phases in a liquid hydrocarbon product, said device comprising:
- a tubular container (1) adapted to contain the mixture of the two phases, said container being provided with a means (3) for measuring the temperature of the mixture and with a hermetically sealing means (2);
- a rigid frame (5) comprising a cavity (4) adapted to receive the container (1) ;
- a means (5,6) for adjusting the temperature of the cavity (4) ;
- means (9) for emitting and means (10) for receiving a light beam, which means are disposed transversely on either side of said cavity, and
- a base (8) intended to support the frame (5), said base being provided with means (11) adapted to impart vibrating movement thereto;
said device being characterised in that the cavity (4) intended to receive the container (1) is inclined at an angle of between 10 and 45°and, preferably, between 20 and 25° relative to the horizontal.

2. A device according to claim 1, characterised in that the tubular container (1) is closed by a metal cap (2) provided with an elastomeric sealing pellet, through which passes a temperature-measuring probe (3)

3. A device according to either of claims 1 and 2, characterised in that the frame (5) comprises a metal block pierced by a tubular recess (4) of complementary shape to that of the container (1).

4. A device according to claim 3, characterised in that the metal block (5) comprises a heating resistance (6) and a cooling circuit (7) in which a heat-transfer fluid circulates.

5. A device according to claim 3, characterised in that the metal block (5) comprises a PELTIER effect heating and cooling system.

6. A device according to any one of claims 1 to 5, characterised in that the optical beam is an infrared radiation beam.
